# FASCICULE DE BREVET EUROPEEN

(11) **EP 0 689 820 B1**
(45) Date de publication et mention de la délivrance du brevet: **12.04.2000**
(21) Numéro de dépôt: 95401570.7
(22) Date de dépôt: 29.06.1995
(51) Int. Cl.: A61F 13/15

(54) **Article d'hygiène féminine muni d'éléments de diffusion des fluides corporels**
Frauenhygienartikel mit Dochtelement für körperliche Flüssigkeiten
Lady's hygiene article provided with means for wicking body fluids

(30) Priorité: 01.07.1994 FR 9408136
(43) Date de publication de la demande: 03.01.1996
(73) Titulaire: FORT JAMES FRANCE, 68320 Kunheim (FR)
(72) Inventeur: Lefebvre du Grosriez, Carol, F-78600 Maisons Lafitte (FR)
(74) Mandataire: David, Daniel

(56) Documents cités:
- EP-A- 0 525 676
- DE-U- 9 201 050
- FR-A- 2 592 896
- NL-A- 8 901 653

## Description

L'invention concerne généralement un article d'hygiène tel qu'une serviette périodique, un article pour l'incontinence ou autres.

Cet article a pour particularité de comporter des éléments de diffusion des fluides corporels améliorant la pénétration de ces fluides dans les parties absorbantes de l'article en éloignant le flux du centre de la serviette, et pouvant créer, suivant leur emplacement, des barrières latérales et/ou longitudinales aux fluides.

Dans le domaine de l'hygiène féminine, les protections externes classiques actuellement sur le marché ont pour défaut rhédibitoire d'entraîner des fuites notamment latérales qui endommagent et salissent les sous-vêtements. En effet, une protection périodique classique fixée par adhésif sur un sous-vêtement n'est pas en contact permanent avec le corps de l'utilisatrice, notamment lorsque celle-ci se déplace ou change de position.

Un autre problème pouvant conduire aux fuites latérales dans les protections actuelles qui comportent ou non des agents superabsorbants, est la saturation de la région centrale d'absorption des protections. Cette région centrale correspond à la zone de réception des fluides (ou encore zone "d'impact" du flux sur la protection). En effet, une fois cette région saturée, même si toute la capacité d'aborption de la serviette périodique n'a pas été utilisée, les fluides s'écouleront sur les côtés de la serviette, provoquant des fuites alors que les régions absorbantes situées aux extrémités de celle-ci resteront inutilisées.

Quand l'utilisatrice est en position allongée, les fuites surviennent à l'avant ou à l'arrière de la serviette, du fait du mauvais placement de la serviette qui se décale par rapport au corps de la femme ou encore du fait de la forme de la serviette qui est prévue de préférence pour une utilisation sensiblement horizontale.

Des enquêtes ont rapporté que quinze à cinquante pour cent des femmes portant des serviettes périodiques, se plaignaient de fuites. L'absence de fuite et l'absorption sont les principaux critères de choix des femmes pour leurs serviettes.

Pour tenter de surmonter ces problèmes d'étanchéité et plus particulièrement d'étanchéité latérale, des perfectionnements ont été apportés sur les serviettes périodiques de type classique, c'est-à-dire du type comprenant un matelas absorbant les fluides, une couche supérieure perméable située sur le matelas absorbant et enveloppant, le cas échéant, ce matelas, et une couche inférieure imperméable située sous le matelas absorbant.

Le brevet européen n° 0 91 412 décrit une serviette comportant des moyens élastiques fixés sur les bordures latérales de la serviette afin de soulever celles-ci le long du matelas, de provoquer la contraction d'au moins la partie centrale de la bordure et de retenir ces bordures dans leurs positions relevées afin de former des barrières latérales contre les fuites latérales.

Le brevet européen n° 0 134 086 a pour objet une serviette hygiénique munie de volets latéraux qui, lorsqu'ils sont repliés sous le sous-vêtement, forment des joints ou barrières d'étanchéité au niveau de leurs axes de flexibilité.

La demande de brevet européen n° 0 520 884 révèle un article où des bourrelets latéraux sont formés sur au moins une partie de la zone d'entrejambe. Dans un mode préféré de réalisation, la feuille imperméable constituant la face inférieure du matelas absorbant, est repliée longitudinalement et forme une doublure imperméable du bourrelet, améliorant ainsi l'étanchéité latérale.

Par ailleurs, une recherche de confort et de maintien a conduit au choix de formes dites anatomiques, c'est-à-dire "en sablier" ou rétrécies à l'entrejambe, qui épousent davantage le corps de la femme et permettent un meilleur positionnement de la serviette.

Cependant, ces solutions, pour éliminer les fuites latérales, ne sont que partielles.

D'autres recherches ont été orientées sur des articles d'hygiène féminine consistant en une protection externe munie d'une zone centrale absorbante protubérante qui vient se placer entre les lèvres de la vulve. Ce type d'article tout en étant externe, présente une zone semi-interne assurant ainsi un contact permanent entre le corps de l'utilisatrice et la partie externe de la protection. Des exemples de produits sont illustrés par un grand nombre de brevets dont le brevet américain n° 4 046 147, le brevet européen n° 0 162 451 et le brevet français n° 2 653 328. Cependant, ce genre d'article non seulement est inconfortable mais provoque parfois une rétention excessive des fluides dans la protubérance c'est-à-dire la zone semi-interne de l'article.

On retrouve les problèmes rencontrés avec les protections internes du type tampon, c'est-à-dire l'absorption excessive et la saturation du tampon absorbant qui provoquent de manière réversible des fuites.

Enfin, un autre axe de recherche a été développé pour munir les articles d'hygiène d'une couche de transfert ou de diffusion à base de fibres, destinée à améliorer le drainage et la pénétration des fluides dans les parties absorbantes de l'article.

Le brevet américain n° 3 665 922 révèle une serviette périodique qui, de par sa structure, reçoit immédiatement les fluides qui se déchargent, les transporte rapidement en les éloignant de leur point de décharge et les retient de manière efficace dans sa partie interne absorbante, tout en laissant la face externe supérieure de la serviette relativement sèche. De ce fait, les fuites latérales diminuent.

Plus précisément, ce brevet décrit une serviette périodique dont la couche de revêtement externe formant enveloppe comprend une multiplicité de fibres hydrophobes bouclées individuellement et constituant une couche de non-tissé de type "high loft". Chaque boucle est constituée d'une fibre monobrin. Les extrémités de chaque boucle sont fixées dans la base du non-tissé au moyen d'une couche adhésive.

Bien que cette couche superficielle de fibres améliore le drainage et l'aspect sec de la surface de l'article située côté corps, elle n'assure le transport que de manière localisée ; les fibres ne traversent pas la structure interne de la serviette et ne pénètrent pas dans le matelas.

Le brevet américain n° 3 967 623 décrit généralement une couche de fibres et plus précisément de poils, qui se situe sur la face extérieure d'un article d'hygiène tel qu'une serviette hygiénique, au-dessus d'un voile perforé. Cette couche peut être obtenue par fibrillation à chaud d'une feuille de polymères thermoplastiques.

Dans un mode de réalisation spécifique, ce matériau formant une couche de poils est présent non seulement à la surface de l'article comme couche de revêtement mais également comme couche interne entre le voile perforé et le matelas absorbant, les poils étant tournés vers le matelas. Les fibres individuelles de cette couche interne s'entremêlent avec les fibres individuelles du matelas et créent des canaux dans le matelas où les fluides circulent.

Ainsi, la couche interne dont les fibres ont été traitées par un agent tensio-actif et devenant ainsi hydrophiles en surface, a pour fonction de transporter les fluides vers le matelas.

De même ici, les couches de fibres ou poils n'assurent le transport des fluides que de manière localisée. Les fibres ne traversent pas l'article d'hygiène de sa face supérieure côté corps jusqu'à la face inférieure du matelas. De plus, dans le cas d'une décharge importante de fluides, la couche superficielle de fibres ou poils est si dense que les fluides vont avoir tendance à s'écouler sur la surface des poils sans pouvoir pénétrer dans ce revêtement, ce qui peut provoquer en conséquence des fuites latérales.

Un autre brevet américain n° 4 360 022 décrit une serviette périodique comprenant une couche de fibres hydrophobes disposées longitudinalement sur toute la surface du matelas absorbant, qui est recouverte par une couche de surface ou de couverture formée par l'enveloppe de la serviette.

Ces fibres permettent de guider ou drainer les liquides le long de toute la surface du matelas absorbant mais comme elle ne traversent pas la structure interne de la serviette, elles n'optimisent pas la pénétration des liquides et par ailleurs ne peuvent pas jouer le rôle de barrières latérales.

D'autres demandes de brevet décrivent des moyens permettant le transport et le drainage des fluides corporels au moyen de fibres.

La demande de brevet internationale WO 93/01780 décrit de manière générale une serviette périodique comprenant un voile de surface perméable aux liquides, une couche inférieure imperméable aux liquides, un matelas absorbant positionné entre le voile et la couche inférieure et une couche de transport comportant des éléments orientés dans une direction Z perpendiculaire au plan formé par la serviette. La partie inférieure de cette couche de transport peut être insérée dans le matelas et la partie supérieure de cette couche peut s'étendre au-dessus de la surface du matelas.

Les éléments constituant cette couche de transport sont des fibres formant capillaires. Plus précisément, elles ont une forme et une section telle, que des canaux se créent sur leur surface externe à l'intérieur d'une même fibre. Ces fibres ont par exemple une section en U ou en H, les canaux étant formés par exemple par l'espace entre les jambes du H. La capillarité à l'intérieur de la couche de transport est assurée par chaque fibre. On ne décrit pas d'arrangement précis des fibres entre elles si ce n'est l'assemblage en touffes qui reste un assemblage au hasard sans positionnement des fibres les unes par rapport aux autres. Les fibres formant capillaires ont un denier variant dans l'intervalle de 10 à 35. Elles sont monobrins.

De manière générale, la couche de transport comportant ces fibres, est disposée sous le voile de surface perméable. Dans deux modes de réalisation précis, certains éléments de la couche de transport dépassent à la surface du voile. Dans un premier cas, certaines fibres passent à travers les perforations du voile et leurs extrémités apparaissent en surface.

Dans un second cas, une fenêtre est découpée dans le voile si bien qu'une partie des fibres est découverte en surface de la serviette périodique.

Par ailleurs, dans tous les modes de réalisation décrits dans cette demande, le gradient de capillarité est assuré pour ce dispositif formant serviette périodique, par un ensemble de plusieurs matériaux distincts, de la surface vers le fond de l'article d'hygiène :
- les perforations du voile, d'un certain diamètre,
- les canaux capillaires intrafibres des fibres monobrins constituant la couche de transport, de diamètre plus faible, et
- les canaux interfibres des fibres du matelas absorbant, de diamètre encore plus faible.

Ce dispositif permet de drainer les fluides déposés à la surface du voile en laissant ce voile sec et de transporter les fluides vers le matelas dans des zones absorbantes insaturées. Mais, il ne peut assurer le drainage rapide par un même matériau assurant la capillarité, d'un point de réception des fluides en surface de l'article jusqu'au fond du matelas.

Les fibres ne sont pas organisées précisément entre elles pour créer un élément de diffusion assurant lui-même le transport des fluides à travers toute la structure interne de la serviette jusqu'au fond du matelas.

De ce fait, des opérations supplémentaires de fabrication sont nécessaires pour renforcer le contact de la couche de transport, et donc des fibres, d'une part avec le voile perforé et d'autre part, avec le matelas absorbant.

Par ailleurs, la couche de transport fait partie intégrante de la structure interne de l'article d'hygiène. Dans un mode de réalisation comportant les touffes disposées sous le voile, celles-ci sont formées en prélevant de la couche de transport située à l'intérieur du matelas absorbant, de la bourre, à travers une fente réalisée en surface du matelas. Dans un autre mode de réalisation, chaque fibre formant capillaire est cousue dans le matelas pour former une bouclette, et le voile est ensuite placé sur le pré-assemblage par tout moyen mécanique tel que par dépôt d'adhésif, et le cas échéant est découpé.

Ces articles d'hygiène munis d'une couche fibreuse de transport sont un net progrès dans l'amélioration du drainage des fluides menstruels sans pour autant pouvoir assurer, de manière certaine, la diffusion des fluides dans différentes directions du matelas, en particulier dans des directions radiales ou longitudinales du matelas, et la formation de barrières latérales.

La présente invention a pour but de favoriser la diffusion des fluides dans toutes les directions du matelas, en particulier dans des directions radiales par rapport au centre de l'article, permettant ainsi d'éloigner le flux de la région centrale, de le distribuer dans tout le matelas absorbant et d'éviter de ce fait le phénomène de saturation.

Elle a également pour but d'améliorer la vitesse de pénétration des fluides corporels dans les articles d'hygiène.

L'invention a en outre pour but de drainer de gros débits d'écoulement de fluides.

L'invention a de plus pour but de créer des barrières latérales aux fluides corporels.

L'invention a encore pour but de fournir un article d'hygiène de structure très simple et réalisable industriellement.

L'invention a aussi pour but de fournir un procédé simple de fabrication d'un article d'hygiène à partir d'un pré-assemblage constitué par au moins deux couches de matériau.

Selon l'invention, l'article d'hygiène tel qu'une serviette périodique comprend une couche de surface perméable aux fluides corporels, un matelas absorbant et une couche imperméable aux liquides.

Suivant une caractéristique essentielle de l'invention, l'article comporte au moins un fil tendu sur sa surface parallèlement au matelas, sur une distance de longueur L définissant un point, et, à ses extrémités, traversant l'article d'hygiène jusqu'à la face inférieure du matelas absorbant.

Selon une caractéristique avantageuse de l'invention, le fil est tendu de préférence dans une direction radiale de l'article, sur la couche de surface.

Selon une autre caractéristique avantageuse de l'invention, l'article comporte au moins deux points, une partie au moins desdits points étant disposée sur la partie centrale de la couche de surface de l'article dans la zone de réception des fluides.

Suivant encore une caractéristique de l'invention, l'article comporte au moins deux points, une partie au moins desdits points étant alignés selon la direction longitudinale près des bordures longitudinales de l'article de manière à former des barrières latérales contre les fuites.

Suivant une autre caractéristique avantageuse de l'invention, le fil est multibrins.

L'invention a également pour objet un procédé de fabrication d'un article d'hygiène.

Selon une caractéristique essentielle, ce procédé consiste à :
- disposer la couche de surface sur le matelas absorbant pour former un pré-assemblage,
- coudre au moins un fil à travers le pré-assemblage de manière à former au moins un point d'une longueur L, sur ladite couche de surface de l'article,
- disposer la couche imperméable aux fluides corporels sous le pré-assemblage après couture, et
- solidariser l'ensemble obtenu par exemple au moyen d'un scellage.

D'autres caractéristiques et avantages de l'invention apparaîtront plus clairement à la lecture de la description détaillée de l'invention qui suit, en référence aux dessins dans lesquels :
- la figure 1 représente une vue de dessus de l'article d'hygiène selon l'invention, suivant un premier mode de réalisation ;
- la figure 2 représente une coupe longitudinale de l'article représenté en figure 1, suivant l'axe II-II ;
- la figure 3 est une coupe transversale de l'article représenté en figure 1, suivant l'axe III-III ;
- la figure 4 illustre en vue de dessus un article d'hygiène selon l'invention suivant une variante du mode de réalisation représenté à la figure 1 ;
- les figures 5a, 5b et 5c illustrent des motifs d'implantation de points en surface de l'article d'hygiène suivant des directions radiales ; et
- la figure 6 représente encore un autre mode de réalisation de l'article, notamment de réalisation de points suivant une technique particulière de couture.

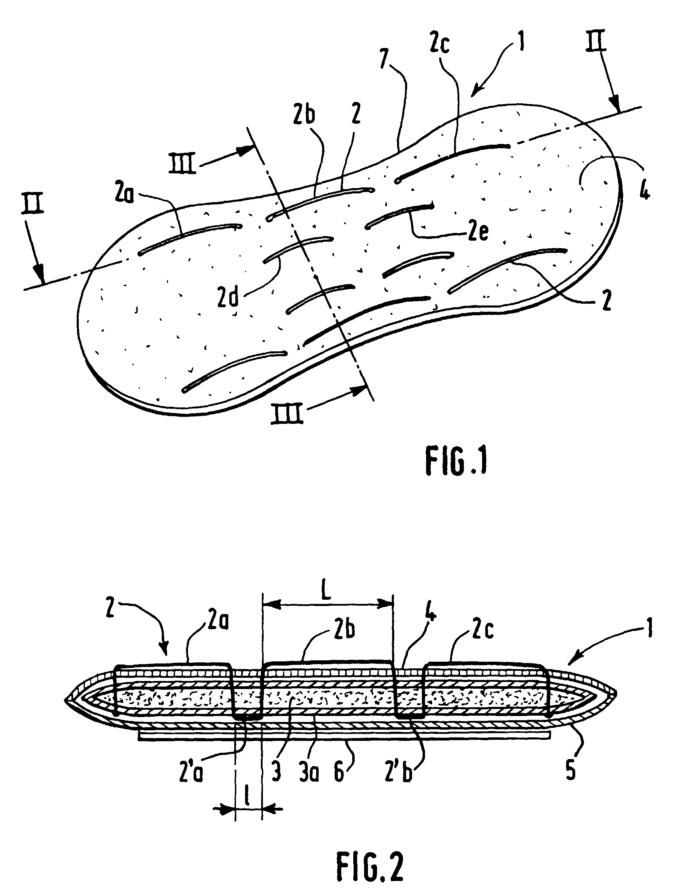

En référence aux figures 1 à 3, l'article d'hygiène selon l'invention, ici une serviette périodique 1, comporte des éléments de diffusion ou de drainage 2 des fluides corporels, dans différentes directions de l'article.

Ces éléments 2 assurent en partie le captage des fluides en surface de l'article et leur drainage ou diffusion dans la structure interne de la serviette pour une meilleure pénétration et absorption des fluides, et, le cas échéant, forment des barrières latérales contre les fuites.

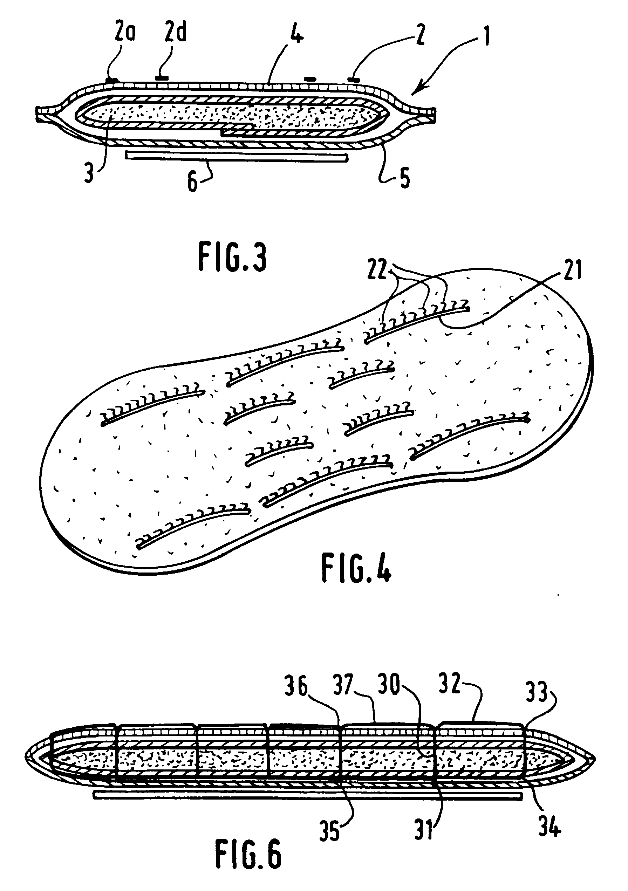

De manière générale, la serviette périodique 1 comprend un matelas absorbant 3, une couche de surface 4 disposée sur le matelas 3 et une couche imperméable 5 située sous le matelas absorbant. Cette couche imperméable peut être disposée entre la face inférieure du matelas et une couche formant enveloppe qui sera au contact du sous-vêtement, ou peut être destinée à être directement en contact avec le sous-vêtement par tout moyen classique de fixation tel qu'un adhésif, comme le représentent les figures 2 et 3. Avant utilisation, cette serviette est munie d'un moyen de protection 6 du moyen de fixation. Si le moyen de fixation est un adhésif, ce moyen peut être une bande siliconée.

Les éléments de diffusion 2 sont constitués par un fil multibrins qui est tendu sensiblement à plat à la surface de l'article sur la couche de surface 4 sur une distance d'une longueur L définissant un point, par exemple 2a, et qui traverse, aux extrémités du point 2a, la serviette périodique 1 jusqu'à la face inférieure 3a du matelas absorbant 3. Ici, plusieurs points 2a, 2b et 2c parallèles au matelas ayant chacun une longueur L, sont formés. Les différents points peuvent être de longueur identique ou différente.

La longueur L des points varie dans l'intervalle allant d'environ 0,5 à environ 6 centimètres et de préférence d'environ 1 à 3 centimètres.

Suivant le mode de réalisation représenté ici en figure 1, les points peuvent être disposés dans la direction sensiblement longitudinale de l'article. Suivant d'autres modes de réalisation, ils peuvent également être disposés dans d'autres directions : transversales ou radiales en surface de l'article.

Les points 2a, 2b et 2c localisés près de la bordure longitudinale 7 de la serviette, permettent de capter et d'arrêter les fluides qui s'écoulent latéralement vers la bordure longitudinale 7 de la serviette et forment ainsi une barrière latérale aux fluides. Les points peuvent également être disposés dans la partie centrale de la serviette, comme l'illustrent les points 2d et 2c, pour drainer et favoriser la pénétration des fluides à partir de la zone de réception des fluides en surface de l'article, vers des zones plus éloignées du centre du matelas.

Les points pourraient également être disposés près des extrémités avant ou arrière de la serviette, notamment près de l'extrémité arrière pour éviter les fuites qui surviennent vers l'arrière lorsque l'utilisatrice est en position allongée sur le dos. Les fils seraient de préférence tendus transversalement au niveau de ces extrémités avant et arrière, c'est-à-dire dans une direction perpendiculaire à l'axe longitudinal de la serviette.

Le fil formant les éléments de diffusion 2 traverse, par couture ou tout autre moyen équivalent, la serviette, et plus précisément le matelas absorbant 3 et la couche de surface 4, de manière à former non seulement des points 2a, 2b et 2c en surface de l'article mais également des points 2'a et 2'b s'étendant sur une longueur l sur le revers, sous le matelas, entre deux lieux d'implantation du fil dans le matelas. La longueur l peut tout aussi bien être supérieure, égale ou inférieure à la longueur L.

Ainsi, les fluides corporels peuvent être collectés à partir des points 2a, 2b et 2c en surface de l'article et drainés dans la structure interne du matelas 3 de manière que les fluides soient diffusés dans des zones du matelas qui ne sont pas directement situées sous la zone de réception des fluides, en particulier vers les bordures longitudinales du matelas ou encore vers les extrémités avant et arrière du matelas.

Dans le mode de réalisation représenté à la figure 4, le fil 21 comporte des fibrilles 22 qui partent du fil dans une direction sensiblement perpendiculaire à l'article d'hygiène en s'éloignant de la surface de l'article. Ces fibrilles favorisent le drainage par capillarité des fluides dans les zones de réception des fluides et, le cas échéant, entrent en contact avec le corps de l'utilisatrice et permettent d'assurer un contact permanent entre l'utilisatrice et le produit. Ce contact permanent est une des principales conditions pour éviter dès l'émission du flux que ce dernier ne sécoule vers les bordures latérales de l'article ou encore aux extrémités avant et arrière de ce même article.

Des études ont en effet montré que le flux a une certaine "attirance" ou "tactisme" pour le derme. Le flux a donc naturellement tendance à rester sur le corps de l'utilisatrice et à s'écouler à partir des points de l'anatomie entrant en contact avec la surface de l'article. Selon la position et l'activité de l'utilisatrice, ces points ou zones de contact varient.

Dans le cas d'un contact "rapproché" entre la protection et l'anatomie, par exemple lorsque le produit est plaqué contre l'anatomie quand l'utilisatrice est en position assise, croise les jambes, etc..., le flux sera immédiatement réceptionné dans la zone centrale de la protection. Les fils tendus parallèlement au matelas draineront le flux et le guideront vers les zones absorbantes non saturées du matelas.

Dans le cas d'un contact "éloigné" entre la protection et l'anatomie, par exemple lorsque l'utilisatrice est debout ou allongée ou porte des sous-vêtements peu ajustés, le flux aura tendance à suivre le profil de l'anatomie et à n'entrer en contact avec la surface du produit que vers ses bordures ou extrémités, ou, dans le cas le plus défavorable, directement avec le sous-vêtement. Cet écoulement du flux le long de l'anatomie favorise les fuites latérales et/ou longitudinales. La présence de fibrilles permet d'assurer un contact permanent entre la protection et l'anatomie. Les fibrilles stoppent l'écoulement du flux vers les extrémités ou bordures du produit, puis le guident vers les fils tendus en surface du produit qui, à leur tour, le diffusent de manière canalisée vers les zones les plus absorbantes du matelas, c'est-à-dire celles qui ne sont pas encore saturées.

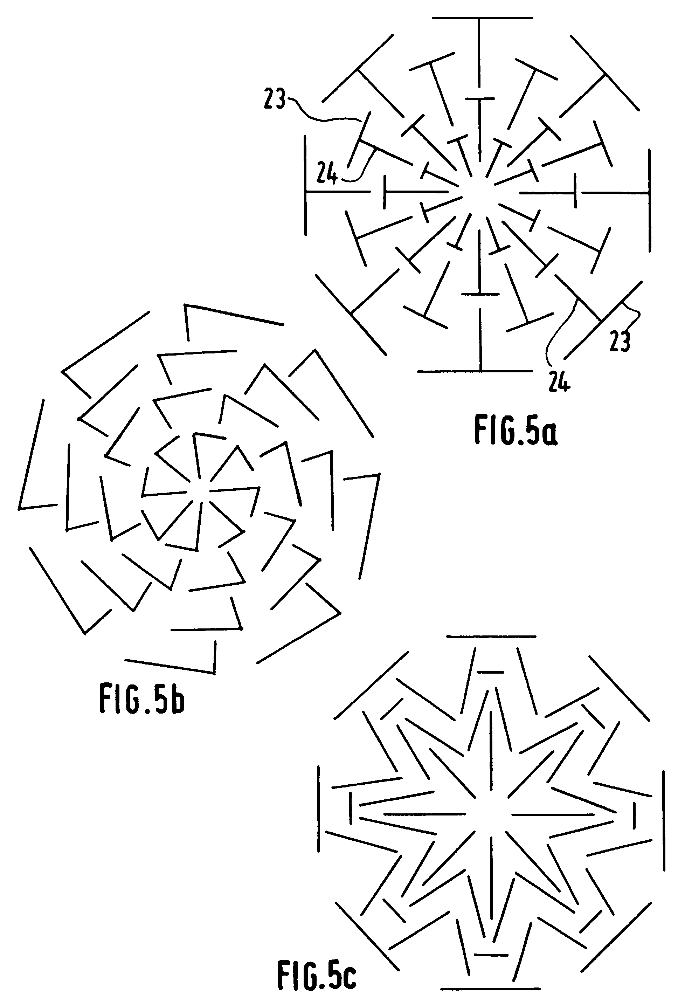

Suivant un mode de réalisation préféré de l'invention, les fils sont tendus dans des directions radiales à partir du centre de l'article, les points pouvant être disposés par exemple sous forme d'étoile. Différents motifs d'implantation des fils sont illustrés aux figures 5a, 5b et 5c. Cette disposition radiale des fils permet d'éloigner le flux de la zone centrale de captage, de le distribuer dans le matelas absorbant et d'éviter ainsi le phénomène de saturation. A cette série de points implantés radialement sur une partie centrale étendue de l'article, peuvent encore s'ajouter des points implantés longitudinalement sur les bordures longitudinales de l'article, formant des barrières latérales et des points implantés latéralement aux extrémités du produit formant des barrières longitudinales.

Pour une protection maximale, cette implantation centrale radiale doit permettre, d'une part, d'éviter au flux de "stagner" en un point central à la surface de l'article et, d'autre part, d'éviter que le flux qui aurait glissé en surface de l'article, sur le voile vers les bordures latérales, sans pénétrer dans le matelas, ne provoque des fuites latérales. En référence aux figures 5a et 5b, pour améliorer l'efficacité de cette implantation, des points 23 supplémentaires sont disposés perpendiculairement et en périphérie des points 24 disposés radialement, pour former des obstacles au flux.

La présente invention de par ses éléments de diffusion assure donc le transport et la répartition des fluides au moyen d'un matériau formé d'un fil passant à travers la structure de l'article comportant la couche de surface et le matelas absorbant. Le ou les fils parcourant l'article remplissent deux types de fonction suivant leur emplacement. En surface de l'article, ils assurent le captage des fluides. Et dans le matelas, lorsqu'ils traversent ce dernier, ils assurent la diffusion des fluides.

La capacité d'absorption de l'article selon l'invention est également augmentée du fait de l'utilisation d'une plus grande partie de la masse absorbante.

Afin d'améliorer l'efficacité du produit, on peut envisager d'avoir des points disposés symétriquement en surface du produit et sous le matelas, le point en surface assurant le captage de fluide et le point symétrique, sous le matelas, diffusant de son côté les fluides. Cette structure de point peut être réalisée au moyen de deux fils distincts cousus simultanément au moyen de deux aiguilles ou encore au moyen d'un seul et même fil cousu dans un sens puis dans l'autre en "aller et retour" en implantant le fil du point "retour" dans les lieux d'implantation du fil du point "aller".

Suivant les différents modes possibles de couture du fil, le fil forme en surface de l'article une ligne continue où les points sont adjacents entre eux ou une ligne interrompue où les points sont espacés les uns des autres.

Pour la fabrication des articles selon l'invention, les matériaux classiquement utilisés, en particulier dans le domaine de l'hygiène féminine : les matériaux des serviettes minces, sont tout à fait transposables au produit selon l'invention.

La couche de surface peut être formée par tout matériau perméable aux fluides : voile plastique perforé, matériau nontissé, cardé ou non, thermolié, etc...

Le matelas absorbant peut être constitué de tout matériau absorbant classique. Dans un mode de réalisation préféré, ce matelas est formé de fibres cellulosiques contenant une matière superabsorbante sous forme de poudre. Par exemple, une bande de fibres cellulosiques, papetières, formée par voie sèche et liée par un latex, dont les deux bords latéraux sont rabattus sur une partie centrale sur laquelle est déposée au préalable le matériau superabsorbant, peut former ce type de matelas. Un tel matelas est représenté en figure 3. Le matériau superabsorbant peut être sous forme de poudre, de feuille et également de poudre liée à des fibres synthétiques. On peut également envisager un matelas constitué d'un mélange de fibres cellulosiques et de fibres synthétiques, placé entre deux couches d'ouate de cellulose, l'ensemble étant thermolié.

D'autres matériaux tels qu'une mousse synthétique, par exemple de polyuréthane, ou naturelle, par exemple de sphaigne traitée, présentant de grandes capacités d'absorption, peuvent être utilisés. Le traitement de la sphaigne mentionnée ci-dessus est décrit dans une demande de brevet européen n° 0 546 585.

La couche imperméable située sous le matelas absorbant est en matériau plastique imperméable, tel que le polyéthylène ou tout matériau équivalent imperméable. Elle peut également être constituée d'un matériau étant à la fois perméable à l'air et à la vapeur d'eau et imperméable aux liquides. Un tel matériau est par exemple un non-tissé de type "meltblown" composé de microfibres, par exemple de polyéthylène. La couche imperméable pourrait encore être constituée d'une mousse de polyuréthane ou tout matériau équivalent.

La couche de surface et la couche imperméable sont associées par exemple par scellage pour envelopper le matelas.

Le matériau formant les éléments de diffusion est maintenant décrit plus en détails.

Il s'agit d'un fil textile multibrins ou filament multibrins continu. Ce fil est de préférence frisé ou texturisé, ce qui améliore sa résilience et permet d'obtenir des points de nature plus souple et plus douce.

Chaque fil comprend entre environ 5 et environ 100 brins. Et chaque brin a un titre d'au moins environ 15 dtex. Le fil a donc un titre d'au moins 75 dtex et de préférence se situant dans l'intervalle allant d'environ 180 à environ 5000 dtex.

Le choix du titre unitaire d'un brin et du nombre de brins par fil peut jouer si un équilibre entre la résilience et la douceur est recherché. Par exemple, pour un brin de titre faible, un grand nombre de brins sera nécessaire pour compenser la faible résilience.

Le fil est de préférence hydrophile mais n'est pas absorbant. Il peut être traité, par exemple par un agent tensioactif, pour le rendre hydrophile.

Le fil peut être à base de fibres naturelles, artificielles, synthétiques, ou leurs mélanges. Les critères de choix de la nature du fil sont à la fois la résilience et la douceur.

Or, compte-tenu du titre élevé des fils utilisés, il est nécessaire que ces derniers soient relativement doux au contact de la peau.

Dans les fibres naturelles, le coton ou le lin peut être utilisé, mais il doit être traité de manière à diminuer son hydrophilie. Dans le cas d'un fil en coton, il s'agit de fils retords, c'est-à-dire de deux ou trois fils associés entre eux par torsion. L'angora peut également convenir.

Dans les fibres artificielles, la viscose est également appropriée si elle est traitée de manière à être moins hydrophile.

Enfin, les fibres synthétiques sont préférées et sont choisies parmi le polyester, le polyamide, le polyéthylène ou le polypropylène. On choisira plus préférentiellement des éléments fibreux en polyamide pour une plus grande douceur, ou en certains polyesters à la fois résilients et doux. Le polypropylène sera choisi si une très grande résilience est recherchée avant tout.

Un exemple de mode de réalisation de l'article selon l'invention est donné ci-après et n'est en aucun cas limitatif.

Dans un premier temps, on forme un pré-assemblage en superposant une couche de surface et un matelas absorbant.

Dans un second temps, on coud un fil à travers ce pré-assemblage pour former, d'une part, en surface de celui-ci du côté orienté vers le corps, au moins un point d'une longueur L, le fil étant implanté dans le préassemblage aux deux extrémités du point, et, d'autre part, sur le revers, sous le matelas, au moins un point, de longueur l, ici inférieure à L. Comme représenté en figure 2, le fil peut être cousu dans une même direction longitudinale. Le fil est piqué jusqu'au fond du matelas et traverse la face inférieure du matelas.

Dans un autre mode de réalisation représenté à la figure 6, où la couture forme une ligne continue sur le revers et une ligne continue sur l'endroit du pré-assemblage, le fil court sous le matelas et diffuse avantageusement les fluides d'un bout à l'autre de ce dernier. En effet, ici, le fil 30 est piqué en un endroit 31 sous le matelas, traverse ce dernier vers la couche de surface, puis forme un point 32 en surface de l'article, traverse à nouveau le pré-assemblage en 33 vers le fond du matelas, court sous le matelas sur la distance séparant les endroits de piquage 34 et 35 pour retraverser le préassemblage en sortant à l'endroit 36 en surface de l'article et former un second point 37 adjacent au point 32 et ainsi de suite.

L'invention comprend tous les moyens complémentaires et équivalents qui n'ont pas été décrits et qui sont à la portée de l'homme du métier.

## Revendications

1. Article d'hygiène féminine (1) tel qu'une serviette périodique, du type comprenant une couche de surface (4) perméable aux fluides corporels, un matelas absorbant (3) et une couche (5) imperméable aux fluides, caractérisé en ce qu'il comporte au moins un fil multibrins (2) tendu sur la surface dudit article (1) parallèlement au matelas (3), sur une distance de longueur L, définissant un point (2a), et, à ses extrémités, traversant l'article d'hygiène jusqu'à la face inférieure dudit matelas absorbant (3).

2. Article selon la revendication 1, caractérisé en ce que le fil est tendu de préférence dans une direction radiale de l'article, sur la couche de surface (4).

3. Article selon la revendication 1, caractérisé en ce que ledit fil est tendu dans la direction longitudinale de l'article, sur la couche de surface (4).

4. Article d'hygiène selon l'une des revendications 1 à 3, caractérisé en ce qu'il comporte au moins deux points, une partie au moins desdits points (2d, 2e), étant disposée sur la partie centrale de la couche de surface (4) de l'article dans la zone de réception des fluides.

5. Article d'hygiène selon l'une des revendications 1, 3 et 4, caractérisé en ce qu'il comporte au moins deux points, une partie au moins desdits points (2a, 2b, 2c) étant alignée selon la direction longitudinale près des bordures longitudinales (17) dudit article de manière à former des barrières latérales contre les fuites.

6. Article selon l'une quelconque des revendications précédentes, caractérisé en ce que chaque fil comprend entre environ 5 et environ 100 brins et en ce que chaque brin unitaire a un titre d'au moins environ 15 dtex.

7. Article selon l'une des revendications 1 à 5, caractérisé en ce que chaque fil a un titre variant de préférence dans l'intervalle allant d'environ 180 à environ 5000 dtex.

8. Article selon l'une des revendications précédentes, caractérisé en ce que le fil est d'origine naturelle telle que le coton, le lin, l'angora, artificielle telle que la viscose ou synthétique, telle que le polyester, le polyamide ou le polypropylène.

9. Article selon l'une des revendications précédentes, caractérisé en ce que le fil est de préférence hydrophile ou rendu hydrophile.

10. Article selon l'une quelconque des revendications précédentes, caractérisé en ce que la longueur L desdits points varie dans l'intervalle allant d'environ 0,5 à environ 6 centimètres et de préférence d'environ 1 à 3 centimètres

11. Article selon l'une quelconque des revendications précédentes, caractérisé en ce que ledit fil (21) est muni de fibrilles (22) s'orientant dans une direction sensiblement perpendiculaire à la surface de l'article.

12. Procédé de fabrication d'un article d'hygiène féminine selon l'une des revendications précédentes, caractérisé en ce qu'il consiste à :
- disposer ladite couche de surface (4) sur le matelas absorbant (3) pour former un pré-assemblage,
- coudre au moins un fil multibrins (2) à travers ledit pré-assemblage de manière à former au moins un point (2a) d'une longueur L sur ladite couche de surface (4) de l'article,
- disposer la couche (5) imperméable aux fluides corporels sous le pré-assemblage après couture, et
- solidariser l'ensemble obtenu par exemple au moyen d'un scellage.

13. Procédé selon la revendication 12, caractérisé en ce que le fil est cousu dans des directions radiales suivant un motif particulier.

## Patentansprüche

1. Erzeugnis (1) für die weibliche Hygiene, wie eine Monatsbinde, von der Art mit einer für die Körperfluids durchlässigen Oberflächenschicht (4), einer absorbierenden Einlage (3), und einer für die Fluids undurchlässigen Schicht (5), dadurch gekennzeichnet, daß es mindestens einen Faden (2) aus mehreren Einzelfäden aufweist, der parallel zu der Einlage (3) auf der Oberfläche des Erzeugnisses (1) über eine Strecke mit der Länge L gespannt ist, die eine Stichlänge (2a) definiert, und der an den Enden dieser Strecke bis zu der Unterseite der absorbierenden Einlage (3) durch das Hygieneerzeugnis hindurchgeht.

2. Erzeugnis gemäß Anspruch 1, dadurch gekennzeichnet, daß der Faden vorzugsweise in einer radialen Richtung des Erzeugnisses auf der Oberflächenschicht (4) gespannt ist.

3. Erzeugnis gemäß Anspruch 1, dadurch gekennzeichnet, daß der Faden in der Längsrichtung des Erzeugnisses auf der Oberflächenschicht (4) gespannt ist.

4. Hygieneerzeugnis gemäß irgendeinem der Ansprüche 1 bis 3, dadurch gekennzeichnet, daß es mindestens zwei Stichlängen umfaßt, wobei mindestens ein Teil der Stichlängen (2d, 2e) auf dem mittleren Teil der Oberflächenschicht (4) des Erzeugnisses in der Aufnahmezone der Fluids angeordnet ist.

5. Hygieneerzeugnis gemäß irgendeinem der Ansprüche 1, 3 und 4, dadurch gekennzeichnet, daß es mindestens zwei Stichlängen umfaßt, wobei mindestens ein Teil der Stichlängen (2a, 2b, 2c) entsprechend der Längsrichtung nahe bei den Längsumrandungen (17) des Erzeugnisses so ausgerichtet ist, daß seitliche Barrieren gegen Lecks gebildet werden.

6. Erzeugnis gemäß irgendeinem der vorhergehenden Ansprüche, dadurch gekennzeichnet, daß jeder Faden zwischen ungefähr 5 und ungefähr 100 Einzelfäden aufweist, und daß jeder Einzelfaden einen Titer von mindestens ungefähr 15 dtex hat.

7. Erzeugnis gemäß irgendeinem der Ansprüche 1 bis 5, dadurch gekennzeichnet, daß jeder Faden einen Titer hat, der vorzugsweise in dem Bereich von ungefähr 180 bis ungefähr 5000 dtex liegt.

8. Erzeugnis gemäß irgendeinem der vorhergehenden Ansprüche, dadurch gekennzeichnet, daß der Faden aus natürlichem Material, wie Baumwolle, Flachs, Angora, oder aus künstlichem Material, wie Viskose, oder aus synthetischem Material, wie Polyester, Polyamid oder Polypropylen ist.

9. Erzeugnis gemäß irgendeinem der vorhergehenden Ansprüche, dadurch gekennzeichnet, daß der Faden vorzugsweise hydrophil ist oder hydrophil gemacht ist.

10. Erzeugnis gemäß irgendeinem der vorhergehenden Ansprüche, dadurch gekennzeichnet, daß die Länge L der Stichlängen in dem Bereich von ungefähr 0,5 bis ungefähr 6 Zentimeter, und vorzugsweise ungefähr 1 bis 3 Zentimeter liegt.

11. Erzeugnis gemäß irgendeinem der vorhergehenden Ansprüche, dadurch gekennzeichnet, daß der Faden (21) mit Fäserchen (22) versehen ist, die sich in einer zu der Oberfläche des Erzeugnisses im wesentlichen senkrechten Richtung orientieren.

12. Verfahren zur Herstellung eines Erzeugnisses für die weibliche Hygiene, gemäß irgendeinem der vorhergehenden Ansprüche, dadurch gekennzeichnet, daß es darin besteht, daß:
- die Oberflächenschicht (4) auf der absorbierenden Einlage (3) angeordnet wird, um ein vorgefertigtes Element zu bilden,
- das vorgefertigte Element mit mindestens einem Faden (2) aus mehreren Einzelfäden so zusammengenäht wird, daß mindestens eine Stichlänge (2a) mit einer Länge L auf der Oberflächenschicht (4) des Erzeugnisses gebildet wird,
- nach dem Nähen die für die Körperfluids undurchlässige Schicht (5) unter dem vorgefertigten Element angeordnet wird, und
- die erhaltene Einheit zum Beispiel mittels einer Versiegelung verfestigt wird.

13. Verfahren gemäß Anspruch 12, dadurch gekennzeichnet, daß beim Nähen der Faden in radialen Richtungen gemäß einem bestimmten Muster angeordnet wird.

## Claims

1. Women's hygiene article (1) such as a sanitary towel, of the type comprising a surface layer (4) permeable to body fluids, an absorbent pad (3) and a layer (5) impermeable to fluids, characterised in that it comprises at least one multi-filament thread (2) held under tension on the surface of said article (1) and parallel to the pad (3), along a distance corresponding to length L, defining a stitch (2a) and, at its extremities, passing through the hygiene article to the lower face of said absorbent pad (3).

2. Article according to Claim 1, characterised in that the thread is held under tension preferably in a radial direction of the article, on the surface layer (4).

3. Article according to Claim 1, characterised in that said thread is held under tension in the longitudinal direction of the article, on the surface layer (4).

4. Hygiene article according to one of Claims 1 to 3, characterised in that it has at least two stitches, one part at least of said stitches (2d, 2e) being arranged in the central part of the surface layer (4) of the article in the fluid-receiving zone.

5. Hygiene article according to one of Claims 1, 3 and 4, characterised in that it has at least two stitches, one part at least of said stitches (2a, 2b, 2c) being aligned according to the longitudinal direction close to the longitudinal edges (17) of said article, so as to form side barriers to prevent leakages.

6. Article according to any of the preceding Claims, characterised in that each thread comprises between approximately 5 and approximately 100 filaments and in that each filament, as a unit, has a count of at least approximately 15 dtex.

7. Article according to one of Claims 1 to 5, characterised in that each thread has a count varying preferably within the range of approximately 180 to approximately 5000 dtex.

8. Article according to one of the preceding Claims, characterised in that the thread is made of natural material, such as cotton, linen, angora, of artificial material, such as viscose, or of synthetic material, such as polyester, polyamide or polypropylene.

9. Article according to one of the preceding Claims, characterised in that the thread preferably is hydrophilic or is rendered hydrophilic.

10. Article according to any of the preceding Claims, characterised in that the length L of said stitches varies within the range of approximately 0.5 to approximately 6 cm and preferably of approximately 1 to 3 cm.

11. Article according to any of the preceding Claims, characterised in that said thread (21) is provided with fibrils (22) oriented in a direction which is substantially perpendicular to the surface of the article.

12. Manufacturing process of a women's hygiene article according to one of the preceding Claims, characterised in that it consists in:
arranging said surface layer (4) on the absorbent pad (3) to form a pre-assembly,
sewing at least one multi-filament thread (2) through said pre-assembly so as to form at least one stitch (2a) of a length L on said surface layer (4) of the article,
arranging the layer (5) impermeable to body fluids under the pre-assembly after the sewing process, and
securing the obtained assembly, for instance through sealing.

13. Process according to Claim 12, characterised in that the thread is sewn in radial directions according to a particular pattern.
